# EUROPÄISCHE PATENTSCHRIFT

(11) **EP 1 799 191 B1**
(45) Veröffentlichungstag und Bekanntmachung des Hinweises auf die Patenterteilung: **24.11.2010**
(21) Anmeldenummer: 05797292.9
(22) Anmeldetag: 27.09.2005
(51) Int. Cl.: A61K 9/00, A61K 47/26

(54) **MODIFIZIERUNG VON OBERFLÄCHEN VON LAKTOSE ALS HILFSSTOFF ZUR VERWENDUNG FÜR PULVERINHALATIVA**
MODIFYING SURFACES OF LACTOSE SERVING AS AN AUXILIARY AGENT TO BE USED FOR POWDER INHALANTS
MODIFICATION DE SURFACES DE LACTOSE UTILISE COMME AUXILIAIRE, POUR DES AGENTS D'INHALATION PULVERULENTS

(30) Priorität: 01.10.2004 DE 102004048389
(43) Veröffentlichungstag der Anmeldung: 27.06.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: TRUNK, Michael, 55218 INGELHEIM (DE); TIMMERMANN, Inga-Lis, 24105 KIEL (DE); MUELLER, Bernd, Wilhelm, 24220 FLINTBEK (DE); STECKEL, Hartwig, Andreas, 24232 SCHOENKIRCHEN (DE)
(74) Vertreter: Hammann, Heinz
(86) Internationale Anmeldenummer: PCT/EP2005/054835
(87) Internationale Veröffentlichungsnummer: WO 2006/037738

(56) Entgegenhaltungen:
- WO-A-01/76560
- WO-A-02/07705
- BUCKTON GRAHAM ET AL: "The use of gravimetric studies to assess the degree of crystallinity of predominantly crystalline powders" INTERNATIONAL JOURNAL OF PHARMACEUTICS (AMSTERDAM), Bd. 123, Nr. 2, 1995, Seiten 265-271, XP002365264 ISSN: 0378-5173
- NAINI V ET AL: "PHYSICOCHEMICAL STABILITY OF CRYSTALLINE SUGARS AND THEIR SPRAY-DRIED FORMS: DEPENDENCE UPON RELATIVE HUMIDITY AND SUITABILITY FOR USE IN POWDER INHALERS" DRUG DEVELOPMENT AND INDUSTRIAL PHARMACY, NEW YORK, NY, US, Bd. 24, Nr. 10, 1998, Seiten 895-909, XP008003519 ISSN: 0363-9045
- DILWORTH S E ET AL: "Approaches to determine the enthalpy of crystallisation, and amorphous content, of lactose from isothermal calorimetric data" INTERNATIONAL JOURNAL OF PHARMACEUTICS, AMSTERDAM, NL, Bd. 284, Nr. 1-2, 11. September 2004 (2004-09-11), Seiten 83-94, XP004580740 online ISSN: 0378-5173
- E.BERLIN AT AL.: "Calorimetric measurement of the heat of desorption of water vapor from amorphous and crystalline lactose" THERMOCHIMICA ACTA, Bd. 2, 1971, Seiten 143-152, XP009060777 Elsevier Publishing Company, Amsterdam
- STECKEL H ET AL: "Calorimetric study on amorphous lactose during and after re-crystallization at different relative humidity" PHARMAZEUTISCHE INDUSTRIE 2005 GERMANY, Bd. 67, Nr. 6, 2005, Seiten 709-712, XP009060674 ISSN: 0031-711X

## Beschreibung

Die Erfindung betrifft ein Verfahren zur Oberflächen-energetischen Passivierung von Lactose, die bei der Herstellung von Inhalationspulvern als Hilfsstoff zum Einsatz gelangen soll, sowie die Verwendung solcher optimierter Trägermaterialien bei der Herstellung von Inhalationspulvern.

### Hintergrund der Erfindung

Pulverinhalativa, wie sie nach dem Stand der Technik hergestellt werden können (beispielsweise DE-A-179 22 07), müssen neben der chemischen Stabilität des Wirkstoffes und der Zusammensetzung des Arzneimittels als solche vor allem auch physikalisch konstante Eigenschaften aufweisen. Darunter versteht man in direkter Art und Weise, dass die Aerosoleigenschaften hinsichtlich ihres aerodynamischen Verhaltens (z. B. aerodynamische Partikelgröße) konstant bleiben. Dies bedingt, dass sowohl für den mikronisierten Wirkstoff als auch für die im Produkt eingesetzten Hilfsstoffe diese in ihrer physiko-chemisch stabilsten Form eingesetzt werden sollten. Für Laktose gilt dabei aus thermodynamischer Sicht, dass dies dann gegeben ist, wenn sie unter Normalbedingungen in Form von alpha-Laktose Monohydrat vorliegt. Dies gilt in besonderem Maße für die kristalline Ordnung der Oberfläche der Partikel.

Es ist bekannt, dass bei der Mahlung von organischen Substanzen durch den starken Eintrag mechanischer Energie amorphe Anteile bzw. Veränderungen der kristallinen Struktur zu thermodynamisch instabileren Modifikationen auftreten können.

Darüber hinaus ist bekannt, dass Lactose in diversen Formen vorkommen kann (Coenraad F. Lerk, Physikalisch-pharmazeutische Eigenschaften von Lactose, Pharmazie in unserer Zeit, 16, 1987, Nr. 2; 39-46).

Beispielsweise während der Mahlung von Laktose oder bei sonstiger mechanischer Belastung entstehende amorphe Laktose kann mittels bekannter technischer Verfahren (z.B. WO 92/18110 oder WO 95/05805) rekristallisiert werden. Diese Verfahren zeigen jedoch den Mangel auf, dass ausschließlich der Abbau amorpher Anteile, wie sie nach Mikronisierverfahren auftreten, erreicht werden kann. Diese Verfahren bedingen jedoch nicht zwingend den Abbau von beta-Laktose, welche bei diesen Konditionierverfahren, sowie bei großtechnischen Mahlungen von Laktose auf einen Korngrößenbereich mit einem Wert x₅₀> 10 µm ebenfalls entstehen können Thermodynamisch instabile Anteile, wie beispielsweise beta-Laktose, stellen auch wenn sie lediglich auf der Oberfläche von Laktose vorhanden sind, einen Faktor dar, welcher beispielsweise die Langzeit- und Lagerstabilität eines Inhalationspulvers negativ beeinflussen kann.

Es ist somit Aufgabe der vorliegenden Erfindung, ein Verfahren bereitzustellen, durch das vorhandene thermodynamisch instabilere Laktose-Anteile, insbesondere Anteile von beta-Laktose, auf der Oberfläche kristalliner Laktose in alpha-Laktose umgewandelt werden können

Die Bestimmung von beta-Laktose, beispielsweise auf der Oberfläche kristalliner Laktose, ist insbesondere bei geringen Anteilen der beta-Laktose analytisch anspruchsvoll.

Der vorliegenden Erfindung liegt daher ferner die Aufgabe zugrunde, ein analytisches Verfahren bereitzustellen, welches die Bestimmung geringster Anteile von beta-Laktose auch in einem überwiegend aus alpha-Laktose bestehendem Laktose-Kristallisat erlaubt.

### Beschreibung der Erfindung

Überraschenderweise wurde gefunden, dass der Rekristallisationsprozess von an Laktosepartikeln oberflächlich anhaftenden thermodynamisch labilen Laktosemodifikationen so gestaltet werden kann, dass bevorzugt Laktose in Form des thermodynamisch stabilen alpha-Laktose-Monohydrats gebildet wird.

Dementsprechend betrifft die vorliegende Erfindung ein Verfahren zur Bereitstellung von ausschließlich alpha-Laktose-Monohydrat enthaltender Laktose, dadurch gekennzeichnet, dass eine nicht ausschließlich alpha-Laktose-Monohydrat enthaltende Laktosemischung einer relativen Luftfeuchte von wenigstens 85% r.F.

für einen Zeitraum von wenigstens 4 Stunden bei einer Temperatur von 20°C bis 40°C ausgesetzt wird und dass nach dem Konditionierschritt die Laktose einem Trocknungsschritt bei einer Luftfeuchte kleiner 20% r.F. unter Erhöhung der Temperatur für einen Zeitraum von mindestens 1 Stunde ausgesetzt wird, wobei die erhaltene alpha-Laktose-Monohydrat enthaltende Laktose eine Desorptionswärme kleiner 60 J/g aufweist.

Unter Laktosemischungen werden im Rahmen der vorliegenden Erfindung Laktosemischungen verstanden, die nicht ausschließlich alpha-Laktose-Monohydrat enthalten. Insbesondere sind solche Laktosemischungen Laktosen, die ungeordnete Kristallstrukturen an der Oberfläche aufweisen. Solche Laktosemischungen fallen beispielsweise an, wenn Laktose einem Mahlprozess unterworfen wird. In einem solchen Mahlprozess wird durch den Eintrag von Energie eine gegebenenfalls vorher vorhandene eindeutig definierte Kristallstruktur der Laktose zerstört, sodass es zum Auftreten von unterschiedlichen, teils amorphen Modifikationen der Laktose kommt. Ferner werden unter Laktosemischungen im Sinne der vorliegenden Erfindung Laktosen verstanden, die Laktose in Form der beta-Laktose enthalten.

Gänge Laktosemischungen, die in das erfindungsgemäße Verfahren eingesetzt werden sind beispielsweise auch solche, die von einer Vielzahl von Herstellen kommerziell angeboten werden. Zu nennen sind hier beispielsweise die folgenden gemahlenen Laktose-Qualitäten der Firma Meggle (z.B. Granulac 70, Granulac 140, Granulac 200, Granulac 230, Sorbolac 400), der Firma DMV (z.B. Pharmatose 150M, Pharmatose 200M, Respitose 200M, Pharmatose 350M), oder auch der Firma Borculo (z.B. Lactochem Powder, Lactochem Fine Powder, Lactochem Coarse Powder, Lactochem Regular Powder, Lactochem Extra Fine Powder, Lactochem Super Fine Powder). Beispiele für kommerziell erhältliche, gesiebte Qualitäten, die als Laktosemischungen dem erfindungsgemäßen Verfahren unterworfen werden können, können bezogen werden von der Firma Meggle (z.B. Sacchelac 80, Sperolac 100, Inhalac 70, Inhalac 120, Inhalac 230), von der Firma DMV (z.B. Pharmatose 125, Pharmatose 325M), oder auch von der Firma Borculo (z.B. Lactochem Coarse Crystals, Lactochem Crystals, Lactochem Fine Crystals, Lactochem Extra Fine Crystals).

In einer bevorzugten Ausführungsform des erfindungsgemäßen Verfahrens wird die Laktosemischung der genannten relativen Feuchte für einen Zeitraum von wenigstens 6 Stunden, besonders bevorzugt wenigstens 12 Stunden, ferner bevorzugt wenigstens 18 Stunden ausgesetzt.

Im Rahmen des erfindungsgemäßen Verfahrens erfolgt das Einwirken der Feuchtigkeit bevorzugt bei einer Temperatur von 25-35°C, beispielsweise bei 25°C, 30°C oder 35°C.

In einer bevorzugten Ausführungsform des erfindungsgemäßen Verfahrens, wird die Laktosemischung einer relativen Feuchte von wenigstens 90% r.F., besonders bevorzugt von wenigstens 93% r.F. ausgesetzt.

Unter relativer Feuchte (r.F.) wird in Übereinstimmung mit dem Stand der Technik auch im Rahmen der vorliegenden Erfindung der Quotient des Partialdruckes des Wasserdampfs und dem Dampfdruckes des Wassers bei der betreffenden Temperatur verstanden.

In bevorzugten Ausführungsformen des erfindungsgemäßen Verfahrens wird ferner so vorgegangen, dass
a) die Laktosemischung derart bereitgestellt wird, dass eine möglichst große Pulverbett-Oberfläche ausgebildet wird,
b) die Laktosemischung sodann den Bedingungen des Konditionierungstschritts gemäβ Anspruch 1 ausgesetzt wird;
c) und dann bei dem Trocknungsschritt gemäβ Anspruch 1 eine Reduzierung der relativen Luftfeuchte, welcher das Laktose-Muster ausgesetzt wird, erfolgt.

Bevorzugt wird erst nach Durchführung der Schritte a und b die Pulverbettoberfläche reduziert. Im Verfahrensschritte wird hierbei unter erhöhter Temperatur die relative Luftfeuchte auf einen Wert kleiner 20% r.h. beziehungsweise kleiner 10% r.h. gebracht.

Durch das erfindungsgemäße Verfahren werden insbesondere die Oberflächen der Laktosepartikel derartig modifiziert, dass sich Laktose in Form ihrer unter Normalbedingungen thermodynamisch stabilsten Modifikation, des alpha-Laktose-Monohydrats ausbildet.

Wahrend des Trocknungschritts des erfindungsgemäßen Verfahrens wird überschüssiges Prozesswasser aus dem System entfernt. Dabei kann die Trocknung durch Reduzierung der relativen Feuchte auf kleiner 20%, bevorzugt kleiner 15 % unter Erhöhung der Temperatur (beispielsweise auf 70°C, oder 75°C oder 80°C oder 85°C) in Abhängigkeit einer Prozesszeit von mindestens 1h, bevorzugt mindestens von 2 h erfolgen. In einer Variante kann das zu trocknende Laktoseprodukt auch einer absolut trockenen Umgebung, wahlweise trockenem Stickstoff mit einer Restfeuchte kleiner 5% r.F. ausgesetzt werden, wobei dann Prozesszeiten von mindestens 6 h, bevorzugt mindestens 12 h gewählt werden sollten.

Ein weitere-Aspekt betrifft alpha-Laktose, die nach dem erfindungsgemäßen Prozess erhältlich ist. Diese ist insbesondere gekennzeichnet durch ihre stöchiometrische Zusammensetzung von Wasser und Laktose (= 1:1). Dabei ist das Kristallwasser fest eingebunden und bis zu Temperaturen von 80°C nicht zugänglich. Eine vollständige Abgabe des Kristallwassers ist beispielsweise erst oberhalb von 100°C bis 120°C gegeben. Dagegen sind Anhydrat-Formen der Laktose, wie beispielsweise beta-Laktose oder auch die ANhydrat-Form der alpha-Laktose durch hygroskopisches Verhalten charakterisiert, so dass Wasser welches als Weichmacher den Rekristallisationsprozess beschleunigt, bei einer nachgeschalteten Trocknung aus kristallinen Anhydrat-Formen der Laktose freigesetzt werden kann. Demgegenüber liegt nach Abschluss der erfindungsgemäßen Rekristallisation von Laktosemischungen in alpha-Laktose-Monohydrat das in das Kristallgitter eingebaute Wasser so fest gebunden vor, dass dieses im Rahmen eines Trocknungsschrittes nicht wieder abgegeben wird. Zusätzliches Wasser, welches adsorptiv an alpha-Laktose Monohydrat gebunden ist, wird nicht beobachtet.

Ein weiterer Aspekt betrifft ferner die Verwendung von nach dem erfindungsgemäßen Verfahren erhältlicher alpha-Laktose als Hilfsstoff bei der Herstellung eines wirkstoffhaltigen Inhalationspulvers.

Diese wirkstoffhaltigen Inhalationspulver können beispielsweise nach Verfahren erhalten werden, die im Stand der Technik, wie beispielsweise den Internationalen Patentanmeldungen WO 02/30390, WO 03/017970 oder auch WO 03/017979 beschrieben sind.

Die mittels des erfindungsgemäßen Herstellverfahrens erhältliche alpha-Laktose kann generell als Hilfsstoff zur Herstellung von Inhalationspulvern eingesetzt werden, die Wirkstoffe enthalten, deren Applikation aus therapeutischer Sicht auf inhalativem Wege sinnvoll erscheint. Bevorzugt gelangen hierbei solche Wirkstoffe zur Anwendung, die beispielsweise ausgewählt sind aus der Gruppe bestehend aus Betamimetika, Anticholinergika, Corticosteroiden, PDEIV-Inhibitoren, EGFR hemmern und LTD4-Antagonisten oder zwei- oder dreifach Kombinationen davon, wie beispielsweise Kombinationen von Antcholinergika mit Betamimetika, Corticosteroiden, PDEIV-Inhibitoren, EGFR-hemmern oder LTD4-Antagonisten, oder Kombinationen von Betamimetika mit Corticosteroiden, PDEIV-Inhibitoren, EGFR-hemmern oder LTD4-Antagonisten, oder Kombinationen von Corticosteroiden mit PDEIV-Inhibitoren, EGFR-hemmern oder LTD4-Antagonisten oder Kombinationen von PDEIV-inhibitoren mit EGFR-hemmern oder LTD4-Antagonisten oder Kombinationen von EGFR-hemmern mit LTD4-Antagonisten. Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, C lenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1H-quinolin-2-on, 4-Hydroxy-7-[2- {[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol,1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol 6-Hydroxy-8- {1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl} -4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl} -4H-benzo[1,4]oxazin-3-on, 6-Hydroxy-8-{1-hydroxy-2-[2-(4-isopropyl-phenyl)-1,1-dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxycarbonylamino-3-cyano-5-fluorophenyl)-2-(tert.-butylamino)ethanol, gegebenenfalls in Form ihrer Racemate, Enantiomere, Diastereomere und gegebenenfalls in Form ihrer pharmakologisch verträglichen Säureadditionssalze, Solvate oder Hydrate.

Bevorzugt sind die Betamimetika ausgewählt aus er Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy} -butyl)-benzenesulfoneamide, 5-[2-(5,6-Diethyl-indan-2-ylamino)-1-hydroxy-ethyl]-8-hydroxy-1*H-*quinolin-2-on , 4-hydroxy-7-[2-{[2-{[3-(2-phenylethoxy)propyl]sulphonyl}ethyl]-amino}ethyl]-2(3H)-benzothiazolone,1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol,1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino] - ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika aind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1*H-*quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on,
6-Hydroxy-8-{1-hydroxy-2-[2-(4-hydroxy-phenyl)-1,1-dimethyl-ethylamino]-ethyl} -4H-benzo[1,4]oxazin-3-on, 6-Hydroxy-8-{1-hydroxy-2-[2-(4-isopropyl-phenyl)-1,1 dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl} -6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl} -phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl} -4H-benzo[1,4]oxazin-3-on, 6-Hydroxy-8- {1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl} -4H-benzo[1,4] oxazin-3-on, 6-Hydroxy-8-{1-hydroxy-2-[2-(4-isopropyl-phenyl)-1,1-dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl} -phenoxy)-buttersäure, 8-{2-[2-(3,4-Difluor-phenyl)-1,1 - dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1*H-*quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säurcadditionssalzc der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromalcat, Hydroacctat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen in den erfindungsgemäßen Verfahren bevorzugt Salze zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen und Trospiumsalzen.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt. Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Antcholinergika kommen ferner bevorzugt in Betracht die nachfolgenden Verbindungen:
- 2,2-Diphenylpropionsäuretropenolester-methobromid ,
- 2,2-Diphenylpropionsäurescopinester-methobromid ,
- 2-Fluor-2,2-Diphenylessigsäurescopinester-methobromid ,
- 2-Fluor-2,2-Diphenylessigsäuretropenolester-methobromid ;
- 3,3',4,4'-Tetrafluorbenzilsäuretropenolester-Methobromid ,
- 3,3',4,4'-Tetrafluorbenzilsäurescopinester-Methobromid ,
- 4,4'-Difluorbenzilsäuretropenolester-Methobromid ,
- 4,4'-Difluorbenzilsäurescopinester-Methobromid ,
- 3,3'-Difluorbenzilsäuretropenolester-Methobromid ,
- 3,3'-Difluorbenzilsäurescopinester-Methobromid ;
- 9-Hydroxy-fluoren-9-carbonsäuretropenolester-Methobromid ;
- 9-Fluor-fluoren-9-carbonsäuretropenolester-Methobromid ;
- 9-Hydroxy-fluoren-9-carbonsäurescopinester-Methobromid ;
- 9-Fluor-fluoren-9-carbonsäurescopinester-Methobromid ;
- 9-Methyl-fluoren-9-carbonsäuretropenolesterMethobromid ;
- 9-Methyl-fluoren-9-carbonsäurescopinester-Methobromid ;
- Benzilsäurecyclopropyltropinester-Methobromid ;
- 2,2-Diphenylpropionsäurecyclopropyltropinester -Methobromid ;
- 9-Hydroxy-xanthen-9-carbonsäurecyclopropyltropinesterMethobromid ;
- 9-Methyl-fluoren-9-carbonsäurecyclopropyltropinester-Methobromid ;
- 9-Methyl-xanthen-9-carbonsäurecyclopropyltropinester-Methobromid ;
- 9-Hydroxy-fluoren-9-carbonsäurecyclopropyltropinester-Methobromid ;
- 4,4'-Difluorbenzilsäuremethylestercyclopropyltropinester-Methobromid.
- 9-Hydroxy-xanthen-9-carbonsäuretropenolester-Methobromid ;
- 9-Hydroxy-xanthen-9-carbonsäurescopinester-Methobromid ;
- 9-Methyl-xanthen-9-carbonsäuretropenolester-Methobromid ;
- 9-Methyl-xanthen-9-carbonsäurescopinester-Methobromid ;
- 9-Ethyl-xanthen-9-carbonsäuretropenolester Methobromid ;
- 9-Difluormethyl- xanthen-9-carbonsäuretropenolester -Methobromid ;
- 9-Hydroxymethyl-xanthen-9-carbonsäurescopinester -Methobromid,
gegebenenfalls in Form ihrer Solvate oder Hydrate.

Im Rahmen der vorliegenden Erfindung werden unter Corticosteroiden Verbindungen verstanden, die ausgewählt sind aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9a-Difluoro-17α-[(2-furanylcarbonyl)oxy]-11β-hydroxy-16α-methyl-3-oxo-androsta-1,4-dien-17β-carbothionsäure (S)-fluoromethylester und 6α,9α-Difluoro-11β-hydroxy-16α-methyl-3-oxo-17α-propionyloxy-androsta-1,4-dien-17β-carbothionsäure (S)-(2-oxo-tetrahydrofuran-3S-yl)ester, gegebenenfalls in Form ihrer Racemate, Enantiomere oder Diastercomere und gegebenenfalls in Form ihrer Salze und Derivate, ihrer Solvatc und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, ST-126, Dexamethason, 6α,9α-Difluoro-17α-[(2-fuanylcarbonyl)oxy]-11β-hydroxy-16α-methyl-3-oxo-androsta-1,4-dien-17β-carbothionsäure (S)- fluoromethylester und 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6α,9α-Difluoro-17α-[(2-furanylcarbonyl)oxy]-11β-hydroxy-16α-methyl-3-oxo-androsta-1,4-dien- 17β-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Im Rahmen der vorliegenden Erfindung werden unter PDEIV-Inhibitoren Verbindungen verstanden, die ausgewählt sind aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopcntyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cylopropylmethoxy-4-difluoromethoxyphenyl)cyclohexam-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay- 198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9*H-*pyrazolo[3,4-c]-1,2,4-triazolo[4,3-a]pyridin und 9-Cyclopentyl-5,6-dihydro-7-ethyl-3-(*tert*-butyl)-9*H-*pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE IV-Inhibitor ausgewählt aus der Gruppe bestehend aus Enprofyllin, Roflumilast, Ariflo (Cilomilast), AWD-12-281 (GW-842470), N-(3,5-Dichloro-1-oxo-pyridin-4-yl)-4-difluoromethoxy-3-cyclopropylmethoxybenzamid, T-440, T-2585, Arofyllin, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1 -on, Cis[4-cyano-4-(3-cyclopropylmethoxy-4-difluoromethoxyphenyl)cyclohexan-1-ol], PD-168787, Atizoram, V-11294A, C1-1018, CDC-801, D-22888, YM-58997, Z-15370,9-Cyclopentyl-5,6-dihydro-7-ethyl-3-(2-thienyl)-9*H-*pyraxolo[3,4-c]-1,2,4-triazolo[4,3-a]pyridin und 9-Cyclopentyl-5,6-dihydro-7-ethyl-3-(tert-butyl)-9*H-*pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE IV-Inhibitor ausgewählt aus der Gruppe bestehend aus Roflumilast, Ariflo (Cilomilast), AWD-12-281 (GW-842470), Arofyllin, 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], Atizoram, Z-15370 , 9-Cyclopentyl-5,6-dihydro-7-ethyl-3-(2-thienyl)-9*H-*pyrazolo[3,4-c]-1,2,4-triazolo[4,3-a]pyridin und 9-Cyclopentyl-5,6-dihydro-7-ethyl-3-(*tert*-butyl)-9H pyrazolo[3,4-c]-1,2,4-triazolo[4,3-a]pyridin, wobei Roflumilast, Z-15370 und AWD-12-281 besondere Bedeutung zukommt, gegebenenfalls in Form ihrer Racemate, Enantiomere oder Diastereomere und gegebenenfalls in Form ihrer pharmakologisch verträglichen Säurcadditionssalzc, Solvate und/oder Hydrate.

Unter Säurcadditionssalzen mit pharmakologisch verträglichen Säuren zu deren Bildung die o.g. PDEIV-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden

Im Rahmen der vorliegenden Erfindung werden unter LTD4-Antagonisten Verbindungen verstanden, die ausgewählt sind aus der Gruppe bestehend aus Montelukast, 1-(((R)-(3-(2-(6,7-Difluoro-2-quinolinyl)ethenyl)phenyl)-3-(2-(2- hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclo-propanessigsäure, Pranlukast, Zafirlukast, [2-[[2-(4-tert-Butyl-2-thiazolyl)-5-benzofuranyl]oxymethyl]phenyl] essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507), wobei Montelukast , Pranlukast und Zafirlukast besonders bevorzugt sind, gegebenenfalls in Form ihrer Racemate, Enantiomere oder Diastereomere, gegebenenfalls in Form ihrer pharmakoligisch verträglichen Säurcadditionssalzc sowie gegebenenfalls in Form ihrer Salze und Derivate, ihrer Solvate und/oder Hydrate.

Unter Säurcadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Im Rahmen der vorliegenden Erfindung werden unter EGFR-Hemmern Verbindungen verstanden, die ausgewählt sind aus der Gruppe bestehend aus 4-[(3-Chlor-4-fluorphenyl)amino]-6-{[4-(morpholin-4-yl)-1-oxo-2-buten-1-yl] amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6- {[4-(N,N-dimethylamino)-1-oxo-2-buten-1-yl]amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4- fluor-phenyl)amino]-6-{[4-((*R*)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6- {[4-((*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-morpholin-4-yl)-ethoxy]-7-methoxy-chinazolin, 4-[(3-Chlor-4-fluophenyl)amino]-6-({4-[N-(2-methoxy-ethyl)-N-methyl-amino]-1-oxo-2-buten-1-yl} amino)-7-cyclopropylmethoxy-chinazolin, 4-[(3-Chlor-4-fluorphenyl)amino]-6-{[4-(N,N-dimethylamino)1-oxo-2-buten-1-yl]amino}-7-cyclopentyloxy-chinazolin, 4-[(*R*)-(1-Phenyl-ethyl)amino]-6-{[4-(N,N-bis-(2-methoxyethyl)-amino)-1-oxo-2-buten-1-yl]amino}-7-cyclopropylmethoxy-chinazolin, 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin, 4-[(3-Chlor-4- fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,4-[(3-Chlor-4-fluorphenyl)amino]-6-{[4-(N-cylopropyl-N-methyl-amino)-1-oxo-2-buten-1-yl]amino}-7-cylopentyloxy-chinazolin, 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxyl-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6- {[4-(N,N-dimethylamino)-1-oxo-2-buten-1-yl]amino}-7-ethoxy-chinolin, 4- {[3-Chlor-4-(3-fluor-benzyloxy)-phenyl]amino}-6-(5-{[(2-methansulfonyl-ethyl)amino]methyl} -furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino1-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cylohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy} -7-methoxy-chinazolin, 4-[(3-Chlor-4-fluorphenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan- 1-yloxy}-7-methoxy-chinazolin, 4-[(3-Chlor-4-fluor-phenyl)amino]-6- {trans-4-[(morpholin-4-yl)carbonylamino]-cyclohexan-1-yloxy} - 7-methoxy-chinazolin, 4-[(3-Chlor-4-fluor-phenyl)amino]-6-{trans-4-[(morpholin-4-yl)sulfonylamino]-cyclohexan-1-yloxy} -7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6- {1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-4- {N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4- yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin,4-[(3-Chlor-4-fluorphenyl)amino]-6-(trans-4-ethansulfonylamino-cyclohexan-1-yloxy)-7-methoxy-chinazolin , 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Chlor-4-fluorphenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluorphenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6- {cis-4-[(morpholin-4-yl)carbonylamino]-cyclohexan-1-yloxy} -7-methoxy-chinazolin, 4- [(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy} -7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy} -7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-demethyl-morpholin-4-yl)carbonyl]-piperidin-4-yloxy} -7-methoxy-chinazolin, 4- [(3-Chlor-4-fluor-phenyl)amino]-6-{1-[(2-methyl- morpholin-4-yl)carbonyl]-piperidin-4-yloxy} -7-methoxy-chinazolin, 4- [(3-Chlor-4-fluor-phenyl)amino]-6-{1-[(S,S)-(2-oxa-5-aza-bicyclo[2.2.1]hept-5-yl)carbonyl]-piperidin-4-yloxy} -7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy} -7-methoxy-chinazolin, 4-[(3-Chlor-4-fluor-phenyl)amino]-6-{-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1- yloxy)-7-methoxy-chinazolin, 4-[(3-Chlor-4-fluor-phenyl)amino]-6-[trans-4-(N-methansulfonyl-N-methyl-amino)-cyclohexan- 1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)mcthoxy]-chinazolin, 4-[(3-Chlor-4-fluor-phenyl)amino]-6-(1-methansulfonylpiperidin-4-yloxy)-7-methoxy-chinazolin, 4-[(3-Chlor-4-fluor-phenyl)amino]-6-(1-cyano-piperidin-4-yloxy)-7-methoxy-chinazolin, Cctuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6- { [4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cylopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6- {[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl} amino)-7-cyclopropylmethoxy-chinazolin, 4-[(3-Chlor-4-fluorphenyl)amino]-6- {[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxyethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6- {[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-butem1-yl]amino}-7-[(S)-(tetrahydrofuran-2-yl)methoxy]-chinazolin,4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5- {[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinylphenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazo lin, 4-[(3-Chlor-4-fluor-phenyl)amino]-6-{2-[4-(2-oxo-morpholin4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin,4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylaminocyclohexan-1-yloxy)-7-methoxy-chinazolin, 4-[(3-Chlor-4-fluor-phenyl)amino]-6-(tetrahydropyran-3-yloxy)-7-methoxy-chinazolin,4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluorphenyl)amino]-6- {1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy} -7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6- {1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluorphenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonylpiperidin-4-yloxy)-7-(2-methoxy-ethoxy)-chinazolin , 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy} -7-methoxy-chinazolin, 4-[(3-Chlor-4-fluor-phenyl)amino]-6-{1-[2-(2-oxopyrrolidin-1-yl)ethyl]-piperid in-4-yloxy}-7-methoxy-chinazolin, 4-[(3-Chlor-4-fluor-phenyl)amino]-6- {1-[(morpholin-4-yl)carbonyl]-piperidin-4-yloxy} -7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy}-7-methoxy-chinazolin, 4-[(3-ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6- {1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl- morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6- {1-[(2-methoxyethyl)carbonyl]-piperidin-4-yloxy} -7-methoxy-chinazolin, 4-[(3-Chlor-4-fluor-phenyl)amino]-6- {1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-([4-((R)-6-methyl-2-oxo-morpholin-4-yl)-1-oxo-2-buten-1-yl]amino)-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methy-amino]-1-oxo-2-buten-1-yl}amino)-7-cyclopropylmethoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino)-7-ethoxy-chinolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinylphenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluorphenyl)amino]-6-(tetrahydropyran-3-yloxy)-7-methoxy-chinazolin, 4-[(3-Chlor-4-fluorphenyl)amino]-6- {1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin,
4-[(3-Chlor-4-fluor-phenyl)amino]-6- {1-[(piperidin-1-yl)carbonyl]-piperidin-4-yloxy} -7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-cthoxy)-chinazolin, 4-[(3-Chlor-4-fluor-phenyl)amino]-6-[1-(2-methoxyacetyl)-piperidin-4-yloxy]-7-(2-methoxy-methoxy)-chinazolin, 4-[(3-Ethinyl-phcnyl)amino]-6-(tetrahydropyran-4-yloxy]-7-methoxy-chinazolin, 4-[(3-Chlor-4-fluor-phenyl)amino]-6-(cis-4- {N-[(piperidin-1-yl)carbonyl]-N-methyl-amino}-cyclohexan-1-yloxy)-7-methoxy-chinazolin, 4- [(3-Chlor-4-fluor-phenyl)amino]-6- {cis-4- [(morpholin-4- yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus
- 4- [(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluor-phenyl)amino]-6-{[4-((R)-6-mcthyl-2-oxo-morpholin-4-yl)-1-oxo-2-buten-1-yl]amino}-7-[(S)-(tetrahydrofuran-3-yl)oxy]-chinazolin,
- 4-[(3-Chlor-4-fluor-phenyl)amino]-6-[2-((S)-6-methyl-2-oxo-morpholin-4-yl)-ethoxy]-7-methoxy-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Ethinyl-phenyl)amino]-6,7-bis-(2-methoxy-ethoxy)-chinazolin,
- 4-[(3-Chlor-4-fluorphenyl)amino]-6-{[4-(morpholin-4- yl)-1- -oxo-2-buten-1-yl]amino}-7-[(tetrahydrofuran-2-yl)methoxy]-chinazolin,
- 4-[(3-Ethinyl-phenyl)amino]-6-{[4-(5,5-dimethyl-2-oxo- morpholin-4-yl)-1-oxo-2-bute-1-yl]amino}-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-{1-[(morpholin-4-y)carbonyl]-piperidin-4-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy} -7-methoxy-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexam-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin und
- 4-[(3-Chlor-4-fluor-phenyl)amino]-6-(1-cyano-piperidin-4-yloxy)-7-methoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat,

Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Ein weiterer Aspekt betrifft ferner Inhalationspulver, die neben einem oder mehreren der vorstehend genannten Wirkstoffe als Hilfsstoff das nach dem erfindungsgemäßen verfahren erhältliche alpha-Laktose-Monohydrat enthalten.

Ein weiterer Aspekt betrifft die Verwendung des nach dem erfindungsgemäßen Verfahren erhältlichen alpha-Laktose-Monohydrats zur Herstellung eines Arzneimittels zur Behandlung von Krankheiten, in denen die inhalative Applikation von Wirkstoffhaltigen Arzneimitteln einen therapeutischen Nutzen entfalten kann. Ferner betrifft die vorliegende Erfindung die Verwendung des nach dem erfindungsgemäßen Verfahren erhältlichen alpha-Laktose-Monohydrats zur Herstellung eines wirkstoffhaltigen Inhalationspulvers.

Ein weiterer Aspekt betrifft ein Verfahren zur analytischen Bestimmung von Anteilen von von alpha-Laktose-Monohydrat verschiedenen kristallinen Formen der Laktose sowie gegebenenfalls amorpher Formen der Laktose auf der Partikeloberfläche von alpha-Laktose-Monohydrat-Partikeln. Mittels dieses analytischen Verfahrens kann über die Bestimmung der Desorptionswärme (bzw. Desorptionsenthalpie) beispeislweise auch der Nachweis auf Abwesenheit von beta-Laktose geführt werden. Es hat sich überraschenderweise gezeigt, dass die Messung der Desorptionswärme, welche auftritt, wenn kristalline Laktose hygroskopisch / oberflächlich adsorbiertes Wasser freigibt, als Marker herangezogen werden kann, wie die oberflächlich kristalline Zusammensetzung von Laktose Feststoffpartikeln ausgestaltet ist. Die quantitativ messbare Menge dient hierbei als Bewertungsgröße, ob die Probe hinsichtlich der polymorphen Zusammensetzung der Partikeloberfläche aus alpha-Laktose Monohydrat besteht, oder ob sonstige Anteile kristalliner sowie gegebenenfalls auch amorpher Formen der Laktose auch noch vorhanden sind.

Dementsprechend betrifft ein weiterer Aspekt ferner ein Verfahren zur Bestimmung gegebenenfalls vorhandener Anteile von von alpha-Laktose-Monohydrat verschiedenen kristallinen Formen der Laktose sowie gegebenenfalls amorpher Formen der Laktose auf der Partikeloberfläche von alpha-Laktose-Monohydrat-Partikeln, dadurch gekennzeichnet, dass die Desorptionswärme einer Laktosemischung bestimmt wird.

Ein nach dem erfindungsgemäßen Verfahren erhaltene Laktose ist dadurch gekennzeichnet dass sie eine Desorptionswärme (bzw. Desorptionsenthalphie) kleiner 60 J/g, in bevorzugter Art und Weise kleiner 40 J/g aufweist.

### Experimenteller Teil:

Des erfindungsgemäße Verfahren wurde anhand spezifischer Laktosemischungen erprobt. Eine dieser spezifischen Laktosemischungen wird im Rahmen der vorliegenden Erfindung als "Laktosemix ABA" bezeichnet.. Dabei handelt es sich um eine Mischung aus alpha-Laktosemonohydrat Pharmatose 325M (Hersteller: DMV), beta-Laktose der Qualität Pharmatose DCL21 (Hersteller DMV) und sprühgetrockneter amorpher Laktose (15 %). Die sprühgetrocknete Laktose wurde nach gängigen, im Stand der Technik bekannten Sprühtrocknungsverfahren erhalten.

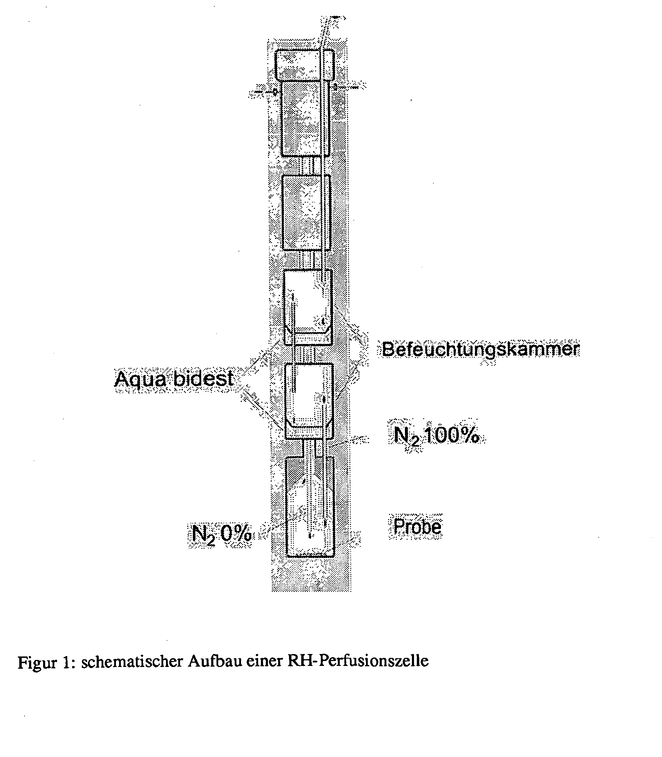

Dic Bestimmung der Dcsorptionscnthalpic wurde für die Laktoscmischung ABA durchgeführt. Die Einwaagen wurden so gewählt, dass der Rekristallisationspeak noch sichtbar war. Zur Bestimmung der Desorptionsenthalpie des Laktosemix ABA, wurden 210 mg eingewogen, entsprechend einem amorphen Anteil von 2,1 mg im einprozentigen Mix, 16,8 mg im achtprozentigen Mix und 31,5 mg im fünfzehnprozentigen Mix. War eine Probe bereits vor der Messung rekristallisiert, konnte nur eine Sorption zu Beginn und eine Desorption zum Ende des Experiments beobachtet werden. Rekristallisierte Proben verhalten sich in der RH-Perfusionszelle (Figur 1) wie kristalline Proben, so dass kein exothermer Rekristallisationspeak und keine endotherme Desorption gemessen werden. Dies hat den Vorteil, dass die integrierte Desorptionsfläche zur Bestimmung der Desorptionsenthalpie nur durch die Desorption des Wassers aus dem rekristallisierten Anteil und nicht durch den Träger entsteht.

Hinsichtlich des analytischen Verfahrens zur Bestimmung von beta-Laktose kann insbesondere wir folgt vorgegangen werden.

Zur Bestimmung der Desorptionsenthalpie einer Probe kann ein Mikrokalorimeter mit Raumfeuchte-Perfusionszelle (im Rahmen dieser Erfindung auch RH-Perfusionszelle bezeichnet) verwendet werden (vgl. Figur 1).

Figur 1 skizziert schematisch den Aufbau einer RH-Perfusionszelle.

Die RH-Perfusionseinheit besteht aus zwei Befeuchtungskammern und einer Stahlampulle, in der die Reaktion abläuft. In die RH-Perfusionseinheit wird trockener Stickstoff (5,0; Messer Griesheim GmbH; Krefeld) eingeleitet und durch ein Flow-Switch-Ventil in zwei Gasströme aufgeteilt. Einer der Gasströme läuft durch die Befeuchtungskammern, die mit 1,5 ml Aqua bidest. (destilliertes Wasser) gefüllt sind und, nachdem der Strom gesättigt ist, in die Stahlampulle (N₂ 100%). Der andere Gasstrom wird außerhalb der Befeuchtungskammern in die Stahlampulle geführt (N₂ O%). Durch Steuerung des Flow-Switch-Ventils über eine Schalteinheit (z.B. ein TAM Acessory Interface 2280 der Firma Thermometric, Schweden) wird je nach programmierter Luftfeuchte die Dauer der Öffnung des Ventils vorgegeben, so dass entweder der feuchte (N₂ 100%) oder der trockene Stickstoffstrom (N₂ O%) in die Stahlampulle gelangt. Durch das Verhältnis trockenem zu feuchtem N₂-Strom, stellt sich in der Stahlampulle die gewünschte relative Luftfeuchte ein. Um die RH-Perfusionseinheit mit Stickstoff zu betreiben, muss der Volumenstrom des Stickstoffs auf 3 ml/min gesenkt werden. Ein Druckminderer (z.B. Typ Hercules), in Kombination mit einem Fcinstdosicrvcntil (z.B. Typ B-SS-2, Firma Swagelok, cv 0,0004) können eingebaut werden, um den Volumenstrom auf diesen geringen Fluss zu reduzieren. Zum Betreiben der RH-Perfisiounszelle wird der Volumenstrom so eingestellt, dass er unterhalb von 3 ml/min liegt.

Die RH-Perfusionszelle wird dabei in die Messzelle eingebracht und es wird so lange gewartet, bis sich das Signal am Mikrokalorimeter der Basislinie wieder nähert. Sobald das Signal einen Wärmefluss (Q) kleiner als 100 µW anzeigt, kann ein Programm für die RH-Perfusionszelle gestartet werden.

Die anzuwendende Methode für die RH-Perfusionszelle besteht aus drei Abschnitten (s. Tabelle 1). Im ersten Abschnitt wird die Stahlampulle klimatisiert, mit trockenem Stickstoff gespült und eine Basislinie aufgezeichnet. Die zweite Pause im ersten Abschnitt stellt sich nur ein, wenn die Basislinie länger als 10 min stabil ist. Innerhalb der Pause wird die RH-Perfusionseinheit aus der Messzelle genommen, die Probe in die Stahlampulle eingewogen und dicht verschlossen. Nach einer Dauer von exakt dreißig Minuten nach Absenken der-RH-Perfusionseinheit in die Messzelle, wird das Experiment manuell weiter geschaltet. Im zweiten Abschnitt wird zunächst für eine Dauer von 240 min die Probe mit einem trockenen Stickstoffstrom (0% r.h.) gespült. Nach der Trocknung erfolgt automatisch innerhalb einiger Minuten der Schritt (Step) von 0% r.h. auf die im Programm festgelegte relative Luftfeuchte. Die Dauer von Main II und Pause II richtet sich nach der Masse des amorphen Anteils der eingewogenen Probe und der Zeit, die die Zelle braucht, um nach der Rekrisiallisation und der Desorption wieder die Basislinie zu erreichen. Im dritten Abschnitt wird die relative Luftfeuchte wieder auf 0% r.h. eingestellt, in dem die Probe nur mit dem trockenen Stickstoffstrom gespült wird. Mit Hilfe einer geeigneten Software (z.B. Digitam Software Version 4.1; Firma Thermometric) werden die aufgezeichneten Daten der Messung in ein geeignetes Rechenprogramm (z.B. Origin Pro G7, OriginLab Corporation, USA) überführt und ausgewertet.

Zur Bestimmung der Desorptionsenthalpie wird der Wärmefluss (Q) (ab 2. Abschnitt, Main I) gegen die Zeit (t) graphisch dargestellt, die Ba sislinie eingezeichnet, subtrahiert und die entstandene Fläche unterhalb der Basislinie integriert. Diese berechnete Wärmeenergie (mJ) wird auf den amorphen Anteil (mg) bezogen, um die Desorptionsenthalpie (J/g) zu berechnen. Dabei hat die Desorptionsenthalpie ein negatives Vorzeichen, da es sich um einen endothermen Prozcss handelt.

**Tabelle1 Ablauf einer Messung mit der RH-Perfusionseinheit**

| Abschnitt | Schritt | Dauer | RH | Stahlampulle |
|---|---|---|---|---|
| 1. | Pause | 20 min | 0% | Leer |
| | Basislinie | 10 min | 0% | Leer |
| | Pause | **--------** | | Einwiegen der Probe |
| 2. | Main I | 90 min | 0% | Trocknen der Probe |
| | Pause I | 150-240 min | 0% | Trocknen der Probe |
| | Main II | Methodenabhängig | Step auf 33%, ,45%, 58%/60% 75%, 80%; 90%; oder 100% ohne Zeitverzögerung | Sorption der Probe |
| | Pause II | Methodenabhängig | bei 33%, ,45%, 60% 75%, 80%; 90%; oder 100% gehalten | Rekristallisation und Desorption des amorphen Anteils |
| 3. | Basislinie | 60 min | Step auf 0% | Desorption der Probe |

## Patentansprüche

1. Verfahren zur Bereitstellung von ausschließlich alpha-Laktose-Monohydrat enthaltender Laktose, **dadurch gekennzeichnet, dass** eine nicht ausschließlich alpha-Laktose-Monohydrat enthaltende Laktosemischung einer relativen Luftfeuchte von wenigstens 85 % r.F. für einen Zeitraum von wenigstens 4 Stunden bei einer Temperatur von 20°C bis 40°C ausgesetzt wird und dass nach dem Konditionierschritt die Laktose einem Trocknungsschritt bei einer Luftfeuchte kleiner 20% r.F. unter Erhöhung der Temperatur für einen Zeitraum von mindestens 1 Stunde ausgesetzt wird, wobei die erhaltene alpha-Laktose-Monohydrat enthaltende Laktose eine Desorptionswärme kleiner 60 J/g aufweist.

2. Verfahren nach Anspruch 1, **dadurch gekennzeichnet, dass** die nicht ausschließlich alpha-Laktose-Monohydrat enthaltende Laktosemischung einer relativen Feuchte von wenigstens 90% r.F. ausgesetzt wird.

3. Verfahren nach einem der Ansprüche 1 oder 2, **dadurch gekennzeichnet, dass** die nicht ausschließlich alpha-Laktose-Monohydrat enthaltende Laktosemischung
a) derart bereitgestellt wird, dass eine möglichst große Pulverbett-Oberfläche ausgebildet wird,
b) dass sie sodann den in Anspruch 1 genannten Bedingungen des Konditionierungschritts ausgesetzt wird;
c) und dann bei dem Trocknungsschritt gemäβ Anspruch 1 ein Reduzierung der relativen Luftfeuchte, welcher das Laktose-Muster ausgesetzt wird, erfolgt.

## Claims

1. Process for preparing lactose which contains exclusively alpha-lactose-monohydrate, **characterised in that** a lactose mixture which does not exclusively contain alpha-lactose-monohydrate is exposed to a relative humidity of at least 85% r.h. for a period of at least 4 hours at a temperature of 20°C to 40°C and **in that** after the conditioning step the lactose is subjected to a drying step at a relative humidity of less than 20% r.h. while the temperature is increased for a period of at least 1 hour, the resulting lactose containing alpha-lactose-monohydrate having a desorption heat of less than 60 J/g.

2. Process according to claim 1, **characterised in that** the lactose mixture which does not exclusively contain alpha-lactose-monohydrate is exposed to a relative humidity of at least 90% r.h.

3. Process according to one of claims 1 or 2, **characterised in that** the lactose mixture which does not exclusively contain alpha-lactose-monohydrate
a) is prepared in such a way as to form the largest possible surface area for the powder bed,
b) it is then exposed to the conditions of the conditioning step recited in claim 1;
c) and then in the drying step according to claim 1 the relative humidity to which the lactose sample is exposed is reduced.

## Revendications

1. Procédé de production de lactose contenant exclusivement du monohydrate de lactose alpha, **caractérisé en ce qu'**un mélange de lactose ne contenant pas exclusivement que du monohydrate de lactose alpha est soumis à une humidité relative de l'air d'au moins 85 % HR pendant une durée d'au moins 4 heures à une température allant de 20°C à 40°C, et **en ce que**, après l'étape de conditionnement, le lactose est soumis à une étape de séchage à une humidité de l'air inférieure à 20 % HR en augmentant la température pendant une durée d'au moins 1 heure, le lactose contenant du monohydrate de lactose alpha obtenu présentant une chaleur de désorption inférieure à 60 J/g.

2. Procédé selon la revendication 1, **caractérisé en ce que** le mélange de lactose ne contenant pas exclusivement que du monohydrate de lactose alpha est soumis à une humidité relative d'au moins 90 % HR.

3. Procédé selon l'une quelconque des revendications 1 à 2, **caractérisé en ce que** le mélange de lactose ne contenant pas exclusivement que du monohydrate de lactose alpha est proposé
a) de telle sorte qu'une surface de lit de poudre la plus grande possible soit formée,
b) qu'il soit soumis ensuite à l'étape de conditionnement dans les conditions citées dans la revendication 1,
c) et ensuite, lors de l'étape de séchage selon la revendication 1, que l'on effectue une réduction de l'humidité relative de l'air à laquelle l'échantillon de lactose est soumis.
